# EUROPÄISCHE PATENTANMELDUNG

(11) **EP 2 353 556 A1**
(43) Veröffentlichungstag der Anmeldung: **10.08.2011**
(21) Anmeldenummer: 11405204.6
(22) Anmeldetag: 21.01.2011
(51) Int. Cl.: A61F 9/06

(54) **Schweisserschutzmaske**

(30) Priorität: 29.01.2010 CH 1082010
(71) Anmelder: OPTREL AG, 9630 Wattwil (CH)
(72) Erfinder: Brändle, Ignaz, 9607 Mosnang (CH)
(74) Vertreter: Frei Patent Attorneys

(57) **Zusammenfassung**

Die Erfindung betrifft eine Schweisserschutzmaske, enthaltend ein Grundtragelement mit einer Sichtöffnung zur Aufnahme eines Filterelementes als Blendschutz. Die Schweisserschutzmaske weist wenigstens einen biegsamen Maskenbereich auf, welcher an das Grundtragelement anschliesst, und der biegsame Maskenbereich ein flächenförmiges, wandartiges Maskenelement beinhaltet oder daraus besteht, welches sich durch die Eigenschaft auszeichnet, dass dieses manuell verformbar ist und sich in unterschiedliche Positionen oder dreidimensionalen Formen biegen lässt.

## Beschreibung

Die Erfindung bezieht sich auf eine Schweisserschutzmaske, enthaltend ein Grundtragelement mit einer Sichtöffnung zur Aufnahme eines optischen Filterelementes als Blendschutz.

### STAND DER TECHNIK

Eine Schweisserschutzmaske, worunter definitionsgemäss auch ein Schweisshelm fällt, umfasst ein Grundtragelement, welches unter anderem eine Sichtöffnung aufweist, in welche in der Regel eine die Sichtöffnung abdeckende Schutzscheibe und ein hinter der Schutzscheibe angeordnetes, optisches Filterelement angeordnet ist. Das Grundtragelement von Schweissmasken oder Schweisshelmen dient neben der tragenden Funktion auch dem Schutz des Schweissers gegen mechanische Einwirkungen und gegen Wärmestrahlung. Das Grundtragelement besteht üblicherweise aus einem Kunststoff, beispielsweise aus Polyamid und wird üblicherweise durch Spritzguss hergestellt. Das Filterelement ist in der Sichtöffnung des tragenden Teils angeordnet und dient dem optischen Schutz des Maskenträgers, es stellt also den eigentlichen Blendschutz der Vorrichtung dar. Das Filterelement kann passiv sein, das heisst, es kann beispielsweise aus Schwarzglas bestehen. Das Filterelement kann auch aktiv sein, das heisst, es sperrt oder reduziert den Lichtdurchgang, wenn die Blendschutzeinrichtung das Licht eines Schweissbogens detektiert. Die Schutzscheibe deckt die Sichtöffnung des tragenden Teils, derart, dass das Filterelement und gegebenenfalls der optische Sensor hinter der Schutzscheibe, das heisst, in getragenem Zustand zwischen der Schutzscheibe und den Augen des Schweissers positioniert sind. Die Schutzscheibe ist transparent und dient dem Schutz des optischen Filterelementes vor Verschmutzung und Beschädigung.

Strahlung stellt für einen Schweisser bekanntlich eine erhebliche gesundheitliche Gefährdung dar. Trotz Schweisserschutzmaske bzw. Schweisshelm mit einem oben beschriebenen optischen Filterelement kann der Fall auftreten, dass Strahlung hinter dem Schweisser reflektiert wird und so von hinten in den Helm gelangt. Durch das Filterelement wird das Licht wiederum zurückgeworfen und kann den Schweisser blenden. Die Problematik besteht vor allem auch dann, wenn die Schweisserschutzmaske nicht optimal der Kopfform des Schweissers angepasst ist und daher nach hinten Öffnungen zwischen Maske und dem Kopf für den Strahlungseinritt ausgebildet werden.

Es besteht zwar die Möglichkeit den Schweisshelm auch am Hinterkopf zu verschliessen, indem eine zusätzliche Lederkappe an den Schutzhelm montiert wird. die Montage und Handhabung sind jedoch nicht anwenderfreundlich. Ausserdem wird durch das Verschliessen die Luftzirkulation im Helm erschwert, woraus im Helminneren eine unangenehme Wärmeentwicklung resultiert.

### DARSTELLUNG DER ERFINDUNG

Der vorliegenden Erfindung liegt daher die Aufgabe zugrunde, die Blendgefahr durch oben genannte Reflexionen durch Verbesserung der bisherigen Schweisserschutzmasken weiter zu minimieren. Ferner soll die Möglichkeit geschaffen werden, die Schweisserschutzmaske individuell der Kopfform der einzelnen Benutzer anzupassen, ohne dass hierzu individualisierte, auf den Benutzer abgestimmte Schweisserschutzmasken als Einzelanfertigungen produziert werden müssen.

Die Aufgabe wird dadurch gelöst, dass die Schweisserschutzmaske wenigstens einen biegsamen Maskenbereich aufweist, welcher an das Grundtragelement anschliesst, und der biegsame Maskenbereich ein flächenförmiges, wandartiges Maskenelement beinhaltet oder daraus besteht, welches sich durch die Eigenschaft auszeichnet, dass dieses manuell verformbar ist und sich in unterschiedliche Positionen oder dreidimensionalen Formen biegen lässt.

Gemäss einer **ersten Ausführungsform** der Erfindung umfasst der biegsame Maskenbereich ein flexibles, biegsames flächenförmiges Maskenelement, welches nicht formstabil zu sein braucht, jedoch formstabil gemäss der nachfolgenden Umschreibung sein kann. Das biegsame Element kann z. B. ein textiles Flächengebilde sein. Das textile Flächengebilde kann z. B. ein- oder beidseitig mit einem Kunststoff beschichtet sein. Die Beschichtung kann ebenfalls durch Hinter- bzw. Umspritzen des textilen Gebildes in einem Spritzgiessverfahren geschehen.

Das Maskenelement lässt sich über eine direkt oder indirekt an der Schweisserschutzmaske, insbesondere am Grundtragelement, angebrachte Stütz- und Arretiereinrichtung in einer bestimmten Positionen oder Formgebung stützen und fixieren. Die Stütz- und Arretiereinrichtung enthält z. B. einen Verstellmechanismus, welcher erlaubt, das biegsame Maskenelement in mehr als einer Stellung bzw. Position oder Formgebung einzustellen und dieses zu fixieren. Da das biegsame Maskenelement nicht formstabil bzw. nicht selbst tragend ist, muss es durch die Stütz- und Arretiereinrichtung einerseits gestützt und andererseits in seiner eingestellten Position bzw. Formgebung gehalten werden. Die Stütz- und Arretiervorrichtung kann z. B. durch ein Kopfband gebildet werden bzw. an diesem befestigt sein. Das Kopfband wiederum ist an der Schweisserschutzmaske, insbesondere am Grundtragelement, angebracht und dient zum Tragen der Schweisserschutzmaske.

Gemäss einer **zweiten Ausführungsform** der Erfindung zeichnen sich die biegsamen Maskenelemente durch die Eigenschaft aus, dass diese manuell plastisch verformbar sind, und sowohl vor als auch nach einem manuellen Umformschritt formstabil sind bzw. bleiben.

Die plastische Verformung oder Plastizität beschreibt die Fähigkeit, fester Stoffe sich unter einer Krafteinwirkung, hier durch manuelle Kraft, zu verformen und diese Form nach der Einwirkung beizubehalten. D.h. insbesondere, dass die Formstabilität der biegsamen Maskenelemente unter dem Einfluss der Schwerkraft und vorteilhaft auch unter dem Einfluss der Gewichtskraft der Schweisserschutzmaske gewährleistet ist. Die Maskenelemente sind also trotz plastischer Umformbarkeit in gewisser Weise selbsttragend und weisen die notwendige Formsteifigkeit auf, um die Maske in der eingestellten geometrischen Form zu halten. Ferner findet praktisch keine Rückfederung der verformten Teile statt, wie dies z. B. bei einer elastischen Verformung der Fall ist. Die plastische Verformung ist dabei insofern reversibel, als dass sich die verformten Bereich wieder in ihre ursprüngliche oder annähernd ursprüngliche Position zurückformen lassen.

Die Maskenelemente können integraler Bestandteil der Schweisserschutzmaske sein. D.h., sie bilden mit dem Grundtragelement eine integrale Einheit. Die Maskenelemente können jedoch auch als eigenständige Elemente ausgebildet sein, welche über entsprechende Verbindungen oder Verbindungskombinationen, wie Formschlussverbindung (z. B. Schnappverbindung, Klippverbindung, Steckverbindung, Nietverbindung, etc.) Kraftschlussverbindung (z. B. Schraubverbindungen, etc.) oder Stoffschlussverbindungen (z. B. Kleben, Löten, Schweissen, etc.) an das Grundtragelement angebracht sind. Die Verbindungen können lösbar oder nicht lösbar sein.

Das Grundtragelement kann ein- oder mehrteilig ausgebildet sein. Es kann sich z.B. aus flächenförmigen, wandartigen Abschnitten zusammensetzen. Zweckmässig bildet das Grundtragelement eine Art schalenförmiges Element aus. Das Grundtragelement ist vorteilhaft nicht plastisch verformbar ausgeführt, sondern weist gegenüber den biegsamen Maskenelementen eine erhöhte Steifigkeit aus. Die Steifigkeit des Grundtragelementes ist bevorzugt so ausgelegt, dass dieses einerseits eine gewisse Stossfestigkeit gegenüber mechanischer Einwirkung aufweist und andererseits eine eingeschränkte plastische oder insbesondere elastische Verformbarkeit zulässt. Das Grundtragelement kann auch als Maskenkörper bezeichnet werden.

Wenn die Schweisserschutzmaske im oberen Bereich, d.h. im Scheitelbereich mehrheitlich oder vollständig geschlossen ist, kann sie auch als Schweisserhelm bezeichnet werden. Das Grundtragelement liegt dann bevorzugt als Helmschale vor.

Grundsätzlich ist es jedoch auch denkbar, dass das Grundtragelement wie die biegsamen Maskenbereiche ebenfalls aus einem biegsamen Material bzw. Materialverbund besteht, welches sich durch die Eigenschaft auszeichnet, dass dieses von Hand plastisch verformbar ist, und sowohl vor als auch nach einem manuellen Umformschritt Formstabilität aufweist. Dabei kann es sich um dasselbe Material bzw. denselben Materialverbund handeln wie bei den biegsamen Maskenbereichen eingesetzt wird. Es kann jedoch auch ein anderes Material bzw. ein anderer Materialverbund verwendet werden. Es kann insbesondere vorgesehen sein, dass das Grundtragelement eine höhere Formstabilität aufweist als der biegsame Maskenbereich, welcher an das Grundtragelement anschliesst, oder dass zur plastischen Verformung des Grundtragelements, z. B. von Hand, höhere Umformkräfte notwendig sind, als beim biegsamen Maskenbereich.

Das Grundtragelement ist bevorzugt so ausgelegt, dass dieses beim Maskenträger wenigstens einen Frontbereich, insbesondere die Augen- und Nasenpartie und gegebenenfalls Teile der oder die komplette Kinn- und Stirnpartie abdeckt. Das Grundtragelement kann aber auch noch andere Gesichts- bzw. Kopfzonen wie Schläfen-, Wangen- oder Scheitelzone des Maskenträgers abdecken.

Im Zusammenhang mit vorliegender Beschreibung wird der Begriff "Material" als Sammelbegriff verwendet, welcher z. B. ein Halbzeug, ein Werkstoff, ein Ausgangsstoff, ein Hilfsstoff oder ein Grundstoff umfassen kann. Ein Material kann überdies in Rohform oder in Vorbearbeitung (Halbzeug), wie z. B. als Textilgebilde, vorliegen. Ferner kann ein Material auch ein Werkstoffverbund darstellen.

Unter dem Begriff Werkstoff sind in diesem Zusammenhang Ausgangsstoffe, wie Metalle, Kunststoffe, Kohle, Glas etc. zu verstehen, aus welchen Erzeugnisse oder Zwischenerzeugnisse (Halbfabrikate) hergestellt werden können. Der Begriff "Material" soll also dem Begriff "Werkstoff' übergeordnet sein.

Das oder die biegsamen Maskenelemente setzen sich bevorzugt aus einer Materialpaarung bzw. einem Materialverbund aus wenigstens zwei Materialien zusammen, bei welchem wenigstens ein erstes Material plastisch verformbar ist und eine formstabilisierende Wirkung auf das Maskenelement ausübt. D.h., das erste Material bewirkt die Formstabilität des Maskenelementes und ist daher selbst auch formstabil. Ein solches Material kann z. B. ein Metall, wie Aluminium oder ein Eisenmetall, wie Stahl bzw. Edelstahl sein, welches in einer entsprechenden Form, z. B. als flächiger Gegenstand, wie Blech oder Folie, oder als Draht bzw. Drahtverbund, mit den entsprechenden Eigenschaften der plastischen Verformbarkeit vorliegt. Die Metallfolie bzw. das Metallblech wie auch ein Drahtverbund (Drahtgeflecht) können hierzu in grossflächiger Form vorliegen.

Das Metallblech bzw. die Metallfolie können zur besseren Umformbarkeit ferner Ausnehmungen wie Löcher bzw. gerichtete Langlöcher (je nach bevorzugter Biegerichtung) enthalten. Das Metallblech bzw. die Metallfolie können z. B. als Lochblech bzw. Lochfolie ausgebildet sein. Ferner können auch Streifen von Metallblechen oder Metallfolien zu einer fachwerkartigen Struktur kombiniert sein, welche zusammen mit wenigstens einem weiteren Material zu einem Materialverbund zusammengeführt werden. Ferner kann die besagte fachwerkartige Struktur auch aus einem Metallblech oder einer Metallfolie herausgearbeitet sein, z. B. durch Stanzen.

Im Weiteren kann das Metallelement auch als Drahtgitter vorliegen, welches ebenfalls mit wenigstens einem weiteren Material zu einem Materialverbund zusammengeführt wird. In den letztgenannten Fällen, bei welchen das flächige Metallelement nicht durchgängig ausgebildet ist, ist der Materialverbund trotzdem zweckmässig ein flächenförmiges, wandartiges und durchgehend geschlossenes Maskenelement, welches insbesondere kein Licht durchlässt. In diesem Fall ist vorzusehen, dass wenigstens ein neben dem oder den Metallelementen im Materialverbund verwendetes zweites Material eine durchgehend geschlossene Fläche ausbildet.

Ein Metallblech bzw. eine Metallfolie, weist z. B. eine Dicke von 0.1 bis 1 mm, vorzugsweise von 0.2 bis 0.5, und insbesondere rund 0.3 mm auf. Ein Metalldraht kann z. B. einen Durchmesser von 0.1 bis 1mm, insbesondere von 0.2 bis 0.5 mm aufweisen.

In einer bevorzugten Weiterbildung der Erfindung umfasst das Maskenelement ein oder mehrere Metallelemente, welches oder welche mit einem verformbaren, vorzugsweise elastisch und/oder plastisch verformbaren Material, wie z.B. einem Kunststoff, einen Materialverbund eingeht oder eingehen. Die Metallelemente können dazu ein oder beidseitig bzw. vollständig mit einem Kunststoff beschichtet sein. Die Kunststoffbeschichtung kann z. B. 0.1 bis 1 mm, insbesondere 0.2 bis 0.5 mm betragen. Der Kunststoff ist bevorzugt ein thermoplastischer Kunststoff, wie z. B. Polyamid, insbesondere PA66, oder ein thermoplastisches Elastomer. Die Metallelemente können vor der Beschichtung mit Kunststoff mit einem Haftvermittler behandelt sein. Die Metallelemente werden bevorzugt mittels Spritzgiessverfahren mit dem Kunststoff hinter- bzw. umspritzt.

Die Metallelemente werden hierzu zweckmässig als Einlegeteile konzipiert, welche danach in einem Spritzgiessverfahren mit Kunststoff hinter- oder umspritzt werden.

Das flexible, biegsame, flächenförmige Maskenelement kann als erstes oder zweites Material gemäss der zweiten Ausführungsordnung auch ein textiles Flächengebilde enthalten. Ist das textile Flächengebilde ein erstes Material, so weist dieses eine entsprechend plastische Verformbarkeit mit Formstabilität wie oben beschrieben auf. Das textile Flächengebilde kann hierzu Fasern aus Metall bzw. Metalldraht enthalten oder daraus bestehen. Ist das textile Flächengebilde ein zweites Material, welches mit einem plastisch verformbaren ersten Material zu einem Materialverbund kombiniert wird, so kann dieses flexibel bzw. biegbar jedoch nicht formstabil sein. Das textile Flächengebilde kann auch ein- oder beidseitig mit Kunststoff beschichtet sein, durch z. B. Hinter- oder Umspritzen in einem Spritzgiessverfahren. Auch hier kann der Kunststoff ein thermoplastischer Kunststoff, wie z. B. Polyamid, insbesondere PA66, oder ein thermoplastische.s Elastomer sein.

Die zur ersten und zweiten Ausführungsform genannten, textilen Flächengebilde können z. B. Gewebe, Gewirke, Gestricke oder Vliese sein. Die textilen Flächengebilde können aus einem oder einer Kombinationen von zwei oder mehreren der folgenden Materialien aufgebaut sein, bzw. diese beinhalten:
- Kunststofffasern;
- Kohlenstofffasern;
- Glasfasern;
- Metallfasern / Metalldraht.

Der biegsame Maskenbereich kann das Grundtragelement abschnittsweise oder vollständig umranden, so dass insbesondere die Abschlusspartie der Schweisserschutzmaske zum Hals bzw. zum Hinterkopf hin wie ein Kragen an die Kontur des Kopfes bzw. Halses anpassbar ist, und auf diese Weise mögliche Eintrittsöffnungen für Strahlung zwischen Maske und Kopf verschlossen werden können. Die Breite der Umrandung bzw. der Randabschnitt kann bis mehrere Zentimeter, insbesondere 0.5 cm bis 15 cm, bevorzugt 1 bis 10 cm betragen. Der biegsame Maskenbereich kann eine Flächengrösse von mehreren Quadratzentimetern, z. B. 1 bis 200 cm², insbesondere 10 bis 100 cm² aufweisen. Die Maskenelemente weisen z. B. eine Gesamtdicke von 0.3 bis 3 mm, insbesondere von 0.5 bis 2 mm, auf.

Dank der biegsamen Maskenbereiche kann der Maskenträger die Form seiner Maske zumindest teilweise individuell nach seinem Wunsch anpassen, indem er nach Belieben die Helmumrandung oder Teilbereiche davon z. B. nach Innen biegen kann, um so den Eintritt von Strahlung von hinten in den Helm zu verhindern.

Die biegsamen Maskenbereiche können eine oder eine Kombination zweier oder mehrere der folgenden Zonen der Schweisserschutzmaske, jeweils einzeln, zusammengesetzt oder als durchgehender Bereich, abdecken:
- Seitenzonen zum Abdecken der Ohrenpartien;
- eine Halszone zum Abdecken wenigstens eines Teils der Halspartie;
- eine Scheitelzone zum Abdecken wenigstens eines Teils des Scheitelbereichs (Kopfoberseite);
- eine Hinterkopfzone zum Abdecken wenigstens eines Teils des Hinterkopfes;
- eine Nackenzone zum Abdecken wenigstens eines Teils des Nackens;
- Kieferzone zum Abdecken wenigstens eines Teiles der Unterkieferpartie;
- Wangenzone zum Abdecken der Wangenbereiche.

Die biegsamen Maskenelemente bzw. Maskenbereiche sind zweckmässig so ausgestaltet und an der Schweisserschutzmaske verformbar angebracht, bzw. mit dieser verbunden, dass keine Strahlung von der Seite oder von hinten in die Maske gelangt. Insbesondere soll auch keine Strahlung durch die Maskenelemente gelangen. Mit dem Begriff "Strahlung" sind vor allem das sichtbare Licht und UV-(Ultraviolett)-Strahlung gemeint. Ferner ist aber auch eine Abschirmung gegenüber IR-(Infrarot)-Strahlung durch das Maskenelement erwünscht. D.h., die Maskenelemente sollen bevorzugt auch gegenüber Wärmestrahlung eine isolierende Wirkung haben.

Sowohl bei der ersten als auch bei der zweiten Ausführungsform empfiehlt es sich unter Umständen, den biegsamen Maskenbereich zu segmentieren und aus mehreren verformbaren Maskenelementen aufzubauen, um auf diese Weise eine Materialverdrängung bei einer grossflächigen Umformung eines Maskenbereichs zu verhindern.

Dank der erfindungsgemässen biegsamen Maskenbereiche weisen die Schweisserschutzmasken, insbesondere Schweisshelme, eine hohe Verformungsfreiheit auf, so dass die Maske den Maskenträger auch gegen Strahlung von hinten optimal abschirmt. Neben der Abschirmung von Strahlung hat die Anpassung der biegsamen Maskenbereiche, insbesondere der biegsamen Randabschnitte der Maske, an die Kontur des Kopfes bzw. Halses auch den Vorteil, dass der Sitz der Maske generell verbessert wird. Die Schweisserschutzmaske kann sogar so ausgebildet sein, dass diese dank dem biegsamen Maskenbereich, insbesondere der biegsamen Umrandung, individuell an die Kopfgeometrie des Maskenträgers angepasst werden kann.

Die biegsamen Maskenbereichen ermöglichen überdies ein enges Anlegen der Schweisserschutzmaske am Kopf, so dass z. B. besser in beengenden Verhältnissen gearbeitet werden kann. Die biegsamen Maskenbereiche können auch dazu vorgesehen werden, um das Tragen eines Gehörschutzes, eines Bauhelmes oder z. B. einer Atemschutzeinheit unter der Schweisserschutzmaske zu ermöglichen. D.h., die biegsamen Maskenbereiche lassen sich entsprechend an die Kontur dieser Geräte anpassen. Ferner können die biegsamen Maskenbereiche auch dazu dienen, die Frischluftzufuhr durch Variieren von Öffnungsweiten individuell einzustellen.

### KURZE BESCHREIBUNG DER ZEICHNUNGEN

Im Folgenden wird der Erfindungsgegenstand anhand von bevorzugten Ausfiihrungsbeispielen, welche in den beiliegenden Zeichnungen dargestellt sind, näher erläutert. Es zeigen jeweils schematisch:
- Fig. 1:: eine erste beispielhafte Ausführungsform einer erfindungsgemässen Schweisserschutzmaske;
- Fig. 2a-2c:: eine zweite beispielhafte Ausführungsform einer erfindungsgemässen Schweisserschutzmaske in verschiedenen perspektivischen Ansichten;
- Fig. 3a-3c:: eine dritte beispielhafte Ausführungsform einer erfindungsgemässen Schweisserschutzmaske in verschiedenen perspektivischen Ansichten;
- Fig. 4a-4i:: verschiedene Ausführungsformen von Einlegeteilen zur Herstellung von biegsamen Maskenelementen gemäss Fig. 3a - 3c;
- Fig. 5a-5b:: eine beispielhafte Ausführungsform einer Stütz- und Arretiereinrichtung für biegsame Maskenelemente an einer Schweisserschutzmaske.

### WEGE ZUR AUSFÜHRUNG DER ERFINDUNG

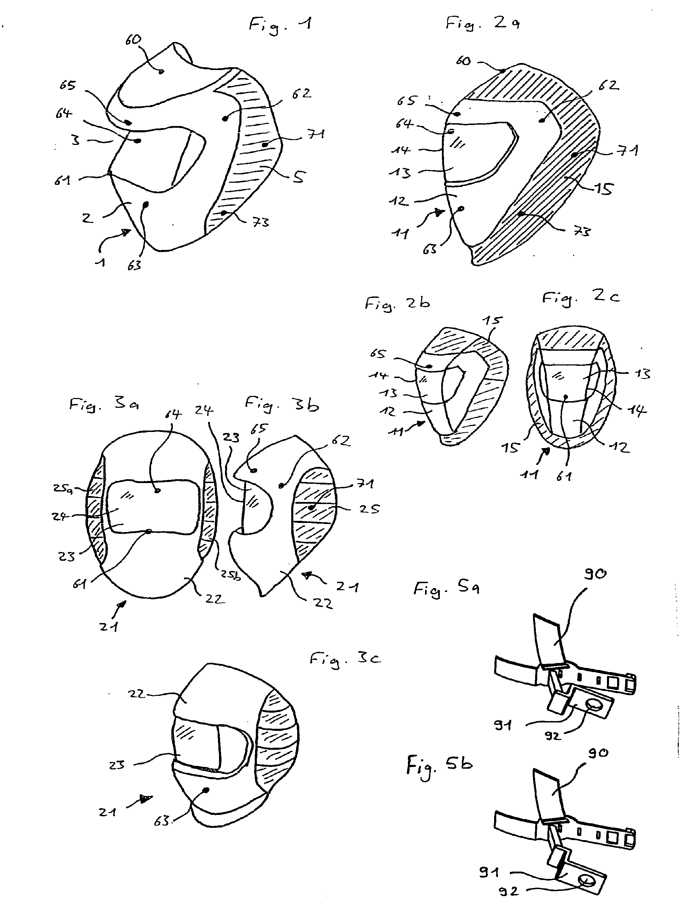

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen. Die in den Fig. 1 bis 4 gezeigten biegsamen Maskenbereiche 5, 15, 25 sind flächenförmige, wandartige Elemente mit einem im allgemeinen Beschreibungsteil beschriebenen Aufbau und mit den ebenfalls in diesem Beschreibungsteil genannten Eigenschaften.

Die Schweisserschutzmaske 1 nach **Figur 1** weist ein Grundtragelement 2 mit einer Sichtöffnung 3 auf. Das Grundtragelement 2 ist so ausgelegt, dass dieses die Scheiteizone 60, die Stirnzone 65, die Nasenzone 61, die Schläfenzone 62, die Kinnzone 63 und durch das Sichtfenster den Augenbereich 64 des Maskenträgers (nicht gezeigt) abdeckt. Im Bereich der Sichtöffnung 3 sind in der Regel eine Schutzscheibe und dahinter ein optisches Filterelement als Blendschutz angeordnet, was jedoch bei der vorliegenden Ausfiihrungsform nicht gezeigt ist. Ferner weist die Schweisserschutzmaske 1 jeweils einen biegsamen Maskenbereich 5 in Form eines Randabschnittes auf, welcher eine Seitenzone 71 zum Abdecken der Ohrenpartien, des Halses und/oder der Unterkieferpartie 73 des Maskenträgers bedeckt. Der biegsame Maskenbereich 5 schliesst an das Grundtragelement 2 an und ist mit diesem lösbar oder nichtlösbar verbunden. Dank dem biegsamen Maskenbereich 5 kann die Spaltöffnung zwischen Hinterkopf bzw. Unterkiefer/Hals und der Maske verschlossen werden, in dem der besagte biegsame Maskenbereich 5 zur Kontur des Kopfes hin verformt wird.

Die Schweisserschutzmaske 11 nach **Figur 2a bis 2c** weist ein Grundtragelement 12 mit einer Sichtöffnung 13 auf. Das Grundtragelement 12 ist so ausgelegt, dass dieses die Stirnzone 65, die Nasenzone 61, die Schläfenzone 62, die Kinnzone 63 und durch das Sichtfenster den Augenbereich 64 des Maskenträgers (nicht gezeigt) abdeckt. Im Bereich der Sichtöffnung 13 ist eine Schutzscheibe 14 und dahinter in der Regel ein optisches Filterelement (nicht gezeigt) als Blendschutz angeordnet. Ferner weist die Schweisserschutzmaske 11 jeweils einen biegsamen Maskenbereich 13 in Form einer Umrandung des Grundtragelementes 12 auf. Die Umrandung bedeckt jeweils eine Seitenzone 71 welche die Ohrenpartien abdeckt, einen Teil der Kinnzone 72, eine Unterkieferpartie 73 sowie einen Teil der Scheitelzone 74 des Maskenträgers. Der biegsame Maskenbereich 15 schliesst an das Grundtragelement 12 an und ist mit diesem lösbar oder nichtlösbar verbunden. Dank dem biegsamen Maskenbereich 15 kann die Öffnung über den Gesamten Randbereich der Maske 11, nämlich zwischen Scheitelzone, Hinterkopf, Unterkiefer, Kinn Hals und der Maske verschlossen werden, in dem der besagte biegsame Maskenbereich 15 zur Kontur des Kopfes hingeformt wird. Der biegsame Maskenbereich 15 umläuft den Randbereich der Maske vollständig und bildet auf diese Weise einen ringartig geschlossenen Maskenbereich aus (siehe Fig. 2c).

Die Schweisserschutzmaske 21 nach **Figur 3a bis 3c** weist ein Grundtragelement 22 mit einer Sichtöffnung 23 auf. Das Grundtragelement 22 ist so ausgelegt, dass dieses die Stirnzone 65, die Nasenzone 61, die Schläfenzone 62, die Kinnzone 63 und über die Sichtöffnung 23 den Augenbereich 64 des Maskenträgers (nicht gezeigt) abdeckt. Im Bereich der Sichtöffnung 23 ist eine Schutzscheibe 24 und dahinter in der Regel ein optisches Filterelement (nicht gezeigt) als Blendschutz angeordnet. Ferner weist die Schweisserschutzmaske 21 jeweils einen biegsamen Maskenbereich 25a, b in Form eines tropfenförmig ausgebildeten Randbereichs am Grundtragelement 22 auf. Es ist jeweils ein biegsamer Maskenbereich für die linke 25b und die rechte 25a Kopfseite vorgesehen. Der biegsame Maskenbereich 25 bedeckt jeweils eine Seitenzone 71, welche die Ohrenpartien des Maskenträgers abdeckt, und schliesst an das Grundtragelement 22 an. Der biegsame Maskenbereich 25 ist mit dem Grundtragelement 22 lösbar oder nichtlösbar verbunden. Der biegsame Maskenbereich 25 weist ferner eine gewölbte, schalenartige Form auf und ist z. B. dazu ausgelegt, die Kapsel eines Kapselgehörschutzes (nicht gezeigt) aufzunehmen. Die Anpassung des biegsamen Maskenbereichs 25 an die Gehörschutzkapsel erfolgt ebenfalls über eine plastische Verformung des biegbaren Maskenbereichs 25.

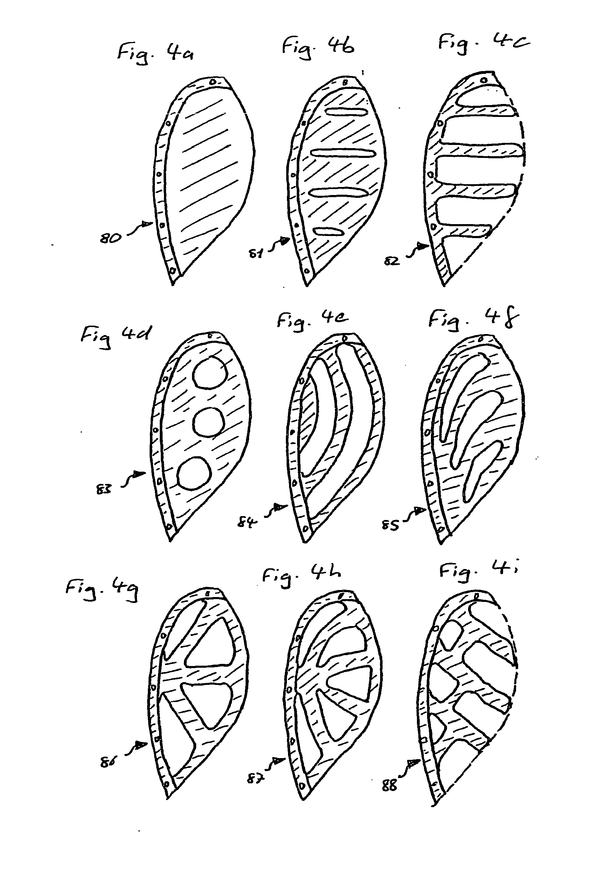

Die **Figuren 4a bis 4i** zeigen verschiedene Ausführungsformen von Einlegeteilen aus einem Metallblech bzw. einer Metallfolie zum ein- oder zweiseitigen Beschichten mit einem Kunststoff und/oder Belegen mit einem textilen Gebilde. Das Beschichten mit Kunststoff kann in einem Spritzgiessverfahren erfolgen. Der Materialverbund, enthaltend ein Einlegeteil der gezeigten Art, ergibt ein von Hand verformbares Maskenelement mit entsprechender Formstabilität wie es zum Beispiel in der Ausführungsform gemäss Figur 3a - 3c Verwendung findet.

Das Einlegeteil 80 gemäss Figur 4a ist als vollflächiges Metallblech bzw. Metallfolie ausgebildet. Gemäss Figur 4b sind im Metallblech bzw. der Metallfolie Langlöcher in Querrichtung zum Einlegeteil 81 eingebracht. Dadurch wird das Einlegeteil 81 bzw. das daraus hergestellte Maskenelement in Langlochrichtung, also in Querrichtung, leichter bieg- bzw. verformbar. Das Einlegeteil 82 gemäss Figur 4c weist fingerförmige, parallel angeordnete Anformungen auf, welche ein zweites Verbundmaterial zu stützen vermögen. Das Einlegeteil 83 gemäss Figur 4d enthält Kreislöcher zur Herabsetzung der Steifigkeit des Einlegeteils. Gemäss Figur 4e sind geschwungene Langlöcher in Längsrichtung des Einlegeteils 84 vorgesehen, welche die Verformbarkeit in dessen Längsrichtung verbessern. Das Einlegeteil 85 gemäss Figur 4f weist diagonal orientierte, kurze geschwungene Langlöcher auf. Die Ausführungsbeispiele gemäss den Figuren 4g, 4h und 4i zeigen verschiedene Formen eines fachwerkartigen Aufbaus der Einlegeteile 86, 87 und 88. Ein fachwerkartiger Aufbau des Einlegeteil verbessert die Verformbarkeit sowohl in Längs- als auch in Querrichtung des Einlegeteils.

Die Ausnehmungen dienen ferner auch der Material- und Gewichtsersparnis. Das zweite Verbundmaterial, z. B. Kunststoff oder ein textiles Gebilde überdecken die Einlegeteile jedoch zweckmässig vollflächig und Übernehmen dabei die Abschirmfunktion.

Die in **Figur 5a und 5b** gezeigte Stütz- und Arretiereinrichtung 91 dient der Befestigung von biegsamen Maskenelementen (hier nicht gezeigt), insbesondere solchen Maskenelementen, welche nicht formstabil sind, an der Schweisserschutzmaske. Die Stütz- und Arretiereinrichtung 91 ist über ein, an dieser Stelle nur ausschnittsweise gezeigtes Kopfband 90 indirekt an der Schweisserschutzmaske (hier nicht gezeigt) befestig. Beim Kopfband handelt es sich um ein herkömmliches Kopfwand wie aus dem Stand der Technik bekannt, welches an der Maske befestigt wird, und über welches die Maske am Kopf getragen wird. Die Stütz- und Arretiereinrichtung 91 kann lös- oder nichtlösbar oder gar integral mit dem Kopfband 90 verbunden sein. Die Stütz- und Arretiereinrichtung 91 weist einen Befestigungsbereich 92, hier eine Befestigungsöffnung, zum Anbringen des flexiblen Maskenelementes (hier nicht gezeigt) auf. Die Stütz- und Arretiereinrichtung 91 lässt sich wenigstens zwischen zwei Positionen (Fig. 5a, 5b) verschieben oder durch Rotation in den genannten Positionen festlegen, so dass sich das flexible Maskenelement in unterschiedliche Positionen bringen und in diesen auch stützen lässt.

### Bezugszeichenliste

- 1: Schweisserschutzmaske
- 2: Grundtragelement
- 3: Sichtöffnung
- 5: Maskenbereich
- 11: Schweisserschutzmaske
- 12: Grundtragelement
- 13: Sichtöffnung
- 14: Schutzscheibe
- 15: Maskenbereich
- 21: Schweisserschutzmaske
- 22: Grundtragelement
- 23: Sichtöffnung
- 24: Schutzscheibe
- 25: Maskenbereich
- 60: Scheitelzone
- 61: Nasenzone
- 62: Schläfenzone
- 63: Kinnzone
- 64: Augenbereich
- 65: Stirnzone
- 71: Seitenzone
- 73: Unterkieferpartie
- 90: Kopfband
- 91: Stütz- und Arretiereinrichtung
- 92: Befestigungsbereich

## Patentansprüche

1. Schweisserschutzmaske, enthaltend ein Grundtragelement mit einer Sichtöffnung zur Aufnahme eines Filterelementes als Blendschutz,
**dadurch gekennzeichnet, dass**
die Schweisserschutzmaske wenigstens einen biegsamen Maskenbereich aufweist, welcher an das Grundtragelement anschliesst, und der biegsame Maskenbereich ein flächenförmiges, wandartiges Maskenelement beinhaltet oder daraus besteht, welches sich durch die Eigenschaft auszeichnet, dass dieses manuell verformbar ist und sich in unterschiedliche Positionen oder dreidimensionalen Formen biegen lässt.

2. Schweisserschutzmaske gemäss Anspruch 1, wobei das biegsame Maskenelement sich durch die Eigenschaft auszeichnet, dass dieses manuell plastisch verformbar, und sowohl vor als auch nach einem manuellen Umformschritt selbsttragend und formstabil ist.

3. Schweisserschutzmaske gemäss Anspruch 1 oder 2, wobei der biegsame Maskenbereich ein flexibles, biegsames flächenförmiges Maskenelement aufweist, welches über eine direkt oder indirekt an der Schweisserschutzmaske, insbesondere am Grundtragelement, befestigte Stütz- und Arretiereinrichtung in einer bestimmten Position oder Formgebung fixier- und stützbar ist.

4. Schweisserschutzmaske gemäss Anspruch 3, wobei die Schweisserschutzmaske ein, bevorzugt am Grundtragelement befestigtes, Kopfband zum Tragen der Schweisserschutzmaske enthält, und die Stütz- und Arretiereinrichtung am Kopfband angebracht ist.

5. Schweisserschutzmaske gemäss einem der Ansprüche 1 bis 4, wobei das flexible, biegsame Maskenelement ein flächenförmiges, textiles Flächengebilde ist oder beinhaltet.

6. Schweisserschutzmaske gemäss einem der vorangehenden Ansprüche, wobei sich das Maskenelement aus einem Verbund von wenigstens zwei Materialien zusammensetzt, wobei wenigstens ein erstes Material plastisch verformbar ist und eine formstabilisierende Wirkung auf das Maskenelement ausübt.

7. Schweisserschutzmaske nach Anspruch 6, wobei das biegsame Maskenelement als erstes Material ein oder eine Anordnung mehrerer Metallelemente umfasst, welches oder welche mit einem flexiblen bzw. verformbaren Material, vorzugsweise einem Material enthaltend oder bestehend aus Kunststoff, als zweites Material einen Materialverbund eingeht oder eingehen.

8. Schweisserschutzmaske gemäss Anspruch 6 oder 7, wobei das biegsame Maskenelement ein oder mehrere ein oder beidseitig mit einem Kunststoff beschichtete Metallelemente, vorzugsweise Metallbleche oder Metallfolien, umfasst.

9. Schweisserschutzmaske gemäss einem der vorangehenden Ansprüche 6 bis 8, wobei das oder die Metallelemente des biegsamen Maskenelements flächige Metallelemente, wie Metallbleche oder Metallfolien, oder Metalldrähte sind.

10. Schweisserschutzmaske gemäss einem der Ansprüche 6 bis 9, wobei der Materialverbund als erstes oder zweites Material ein textiles Flächengebilde umfasst.

11. Schweisserschutzmaske gemäss einem der Ansprüche 5 oder 10, wobei das textile Flächengebilde ein oder eine Kombinationen von zwei oder mehreren der folgenden Materialien umfasst oder daraus besteht:
- Kunststofffasern;
- Kohlenstofffasern;
- Glasfasern;
- Metallfasern / Metalldraht.

12. Schweisserschutzmaske gemäss einem der Ansprüche 5, 10 oder 11, wobei das textile Flächengebilde ein- oder beidseitig mit einem Kunststoff beschichtet ist.

13. Schweisserschutzmaske gemäss einem der vorangehenden Ansprüche, wobei der biegsame Maskenbereich das Grundtragelement wenigstens abschnittsweise, vorzugsweise vollständig, umrandet.

14. Schweisserschutzmaske gemäss einem der vorangehenden Ansprüche, wobei die Schweisserschutzmaske wenigstens eine oder eine beliebige Kombination von zwei oder mehr als zwei der nachfolgenden Zonen aufweist, welche als biegsame Maskenbereiche ausgebildet sind:
- Seitenzonen zum Abdecken der Ohrenpartien;
- eine Halszone zum Abdecken wenigstens eines Teils der Halspartie;
- eine Scheitelzone zum Abdecken wenigstens eines Teils des Scheitelbereichs (Kopfoberseite)
- eine Hinterkopfzone zum Abdecken wenigstens eines Teils des Hinterkopfes;
- eine Nackenzone zum Abdecken wenigstens eines Teils des Nackens;
- eine Kieferzone zum Abdecken wenigstens eines Teils der Unterkieferpartie.
